# EUROPÄISCHE PATENTSCHRIFT

(11) **EP 3 490 625 B1**
(45) Veröffentlichungstag und Bekanntmachung des Hinweises auf die Patenterteilung: **02.09.2020**
(21) Anmeldenummer: 17749412.7
(22) Anmeldetag: 26.07.2017
(51) Int. Cl.: A61L 27/50, A61F 2/18

(54) **VORRICHTUNG MIT STRUKTURIERTER BESCHICHTUNG ZUR VERWENDUNG ALS IMPLANTAT, ZUR BEHANDLUNG VON TROMMELFELLPERFORATIONEN**
DEVICE WITH STRUCTURED COATING FOR USE AS AN IMPLANT, FOR THE TREATMENT OF PERFORATIONS OF EARDRUM
DISPOSITIVE AVEC UNE COUCHE STRUCTURÉ A ÊTRE UTILISÉ COMME UN IMPLANT, POUR LE TRAITEMENT DE PERFORATIONS DE TYMPAN

(30) Priorität: 28.07.2016 DE 102016113956
(43) Veröffentlichungstag der Anmeldung: 05.06.2019
(73) Patentinhaber: Leibniz-Institut für Neue Materialien gemeinnützige GmbH, 66123 Saarbrücken (DE)
(72) Erfinder: ARZT, Eduard, 66123 Saarbrücken (DE); FISCHER, Sarah, 66271 Kleinblittersdorf (DE); KRUTTWIG, Klaus, 66121 Saarbrücken (DE); HENSEL, René, 66128 Saarbrücken (DE); SCHICK, Bernhard, 36145 Hofbieber (DE); WENZEL, Gentiana, 66424 Homburg (DE)
(74) Vertreter: Patentanwälte Gierlich & Pischitzis Partnerschaft mbB
(86) Internationale Anmeldenummer: PCT/EP2017/068872
(87) Internationale Veröffentlichungsnummer: WO 2018/019879

(56) Entgegenhaltungen:
- WO-A1-2007/096082
- WO-A1-2012/054039
- WO-A1-2016/146792
- WO-A2-2010/108003
- DE-A1-102010 026 490
- DE-A1-102012 112 965
- DE-A1-102014 119 470
- US-A1- 2014 329 061
- CARLOS MOTA ET AL: "Multiscale fabrication of biomimetic scaffolds for tympanic membrane tissue engineering", BIOFABRICATION, Bd. 7, Nr. 2, 7. Mai 2015 (2015-05-07), Seiten 1-21, XP055318459, UK ISSN: 1758-5082, DOI: 10.1088/1758-5090/7/2/025005

## Beschreibung

### Gebiet der Erfindung

Die Erfindung betrifft eine Vorrichtung mit einer strukturierten Beschichtung, insbesondere zur Adhäsion auf rauen Oberflächen, vor allem biologischen Oberflächen, wie beispielsweise Trommelfellen.

### Stand der Technik

Die Adhäsion auf rauen Oberflächen ist häufig problematisch. Insbesondere im biologischen Bereich zeigen viele Klebstoffe nur unzureichende Eigenschaften. Gleichzeitig besteht auch das Problem, dass die Oberfläche der Klebstoffe nur unzureichend kompatibel mit biologischen Prozessen, wie beispielsweise Wundheilung ist.

Eine Alternative bieten da trockenadhäsive Oberflächen, wie beispielswiese Geckostrukturen, welche ohne die Vermittlung durch Klebstoffe eine Adhäsion auch an rauen Oberflächen zeigen können. Allerdings sind diese sehr häufig herzustellen und nur beschränkt anpassbar.

DE 10 2014 119 470 A1 offenbart strukturierte Oberflächen mit schaltbarer Adhäsion, wobei die Oberflächen Vorsprünge mit unterschiedlicher senkrechter Höhe aufweisen.

US 2014/0329061 A1 offenbart strukturierte Oberflächen mit einzelnen Vorsprüngen, welche auf ihrer Stirnfläche ebenfalls strukturiert sind.

DE 10 2012 112 965 A1 offenbart eine reversibel strukturierbare Oberfläche, wobei die Vorsprünge durch eine Formgedächtnislegierung gebildet werden.

WO 2012/054039 A1 offenbart eine strukturierte Oberfläche, bei der durch die Strukturierung die Benetzbarkeit gesteuert wird.

WO 2010/108003 A2 beschreibt eine Oberfläche mit single walled nanotubes, welche die Anbindung von Zellen unterstützen.

DE 10 2010 026 490 A1 offenbart ein Verfahren zur Herstellung von strukturierten Oberflächen über ein Prägeverfahren.

WO 2007/096082 A1 offenbart eine Oberfläche mit filamentartigen Strukturen.

Carlos Mota et al. Biofabrication 2015, 7, 025005 beschreibt die Herstellung einer strukturierten Membran als Trommelfellersatz.

WO 2016/146792 A1 beschreibt eine strukturierte Oberfläche mit Vorsprüngen, welche innerhalb eines Vorsprungs Bereiche mit unterschiedlichem Elastizitätsmodul aufweisen, insbesondere mit gekrümmter Grenzfläche.

Trommelfellperforationen sind ein häufig auftretendes Problem, welche zum Hörverlust oder häufig wiederauftretenden Infektionen führen können. Eine häufige Ursache für Trommelfellperforationen können Mittelohrentzündungen, Traumata und postoperative Komplikationen sein. Grundsätzlich kann zwischen akuten, (kleineren) Perforationen, die sich in den meisten Fällen spontan verschließen, und großen oder chronischen Perforationen unterschieden werden. Diese größeren Perforationen benötigen eine operative Versorgung mittels Myringoplastie oder Tympanoplastie, wobei eine hohe Erfolgsquote auftritt, aber neben dem Operationsrisiko auch die Gefahr einer Restperforation besteht. Des Weiteren wird bei der Tympanoplastie autologes Gewebe transplantiert, welches zusätzlich entnommen werden muss. Eines der Hauptprobleme bei der Regeneration von Trommelfellverletzungen stellt eine fehlende Trägerschicht für die Migration von Epithelzellen und die Ausbildung einer trilammelaren Membran dar. Als "Supportplattformen" können generell entweder transplantierte Gewebe, oder Polymere verwendet werden, deren Funktion dann noch mit der Verwendung von Biomolekülen verbessert werden kann. Zu den verwendbaren Polymeren zählen unter anderem Gelatine, Seidenfibroin, Chitosan, Alginate oder Polyglycerolsebacate. Eine aktuelle Übersicht über Ergebnisse bei der Verwendung dieser Polymere und verschiedener Wachstumsfaktoren findet sich in der Übersichtsarbeit von Hong et al. Auch wenn viele der verwendeten Polymere zu hervorragenden Ergebnissen bzgl. des Perforationsverschlusses führen, bestehen bedeutende Unterschiede in der Morphologie des Gewebes.

### Aufgabe

Der Erfindung liegt die Aufgabe zugrunde eine Vorrichtung mit einer strukturierten Beschichtung zur Verwendung bei der Behandlung von Trommelfellperforationen anzugeben, welche eine Adhäsion insbesondere auf rauen, insbesondere auf biologischen Oberflächen aufweist und die Nachteile des Stands der Technik vermeidet.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindungen umfassen auch alle sinnvollen und insbesondere alle erwähnten Kombinationen von unabhängigen und/oder abhängigen Ansprüchen.

Die Aufgabe wird gelöst durch eine Vorrichtung zur Verwendung bei der Behandlung von Trommelfellperforationen mit einer strukturierten Beschichtung, wobei die Vorrichtung eine Trägerschicht umfasst, wobei auf dieser Trägerschicht eine Vielzahl von Vorsprüngen (Pillars) angeordnet ist, die mindestens jeweils einen Stamm mit einer von der Oberfläche wegweisenden Stirnfläche umfassen, dadurch gekennzeichnet, dass mindestens auf der Stirnfläche eine weitere Schicht angeordnet ist, wobei diese Schicht einen anderen Elastizitätsmodul aufweist als der jeweilige Vorsprung.

Diese weitere Schicht bildet auch die Oberfläche der Vorrichtung, welche zur Adhäsion an einer Oberfläche verwendet wird.

In senkrechter Richtung umfasst die Vorrichtung an der Position eines Vorsprungs daher ausgehend von der Trägerschicht mindestens zwei Bereiche mit unterschiedlichem Elastizitätsmodul, nämlich mindestens den Vorsprung und die darauf angeordnete weitere Schicht. Diese weitere Schicht und die Stirnfläche eines Vorsprungs bilden eine Grenzfläche zwischen zwei Bereichen mit unterschiedlichem Elastizitätsmodul. Abhängig von dem Herstellungsverfahren können die Grenzflächen auch dünne Schichten von Verbindungshilfsmitteln umfassen.

Innerhalb eines Bereichs ist der Elastizitätsmodul bevorzugt konstant.

Ein Vorsprung selbst kann auch noch weitere Bereiche aufweisen, welche einen unterschiedlichen Elastizitätsmodul aufweisen.

Die weitere Schicht weist einen geringeren Elastizitätsmodul auf, als der Vorsprung, auf dem sie angeordnet ist. Durch diesen Aufbau wird erreicht, dass der Stamm des Vorsprungs weniger elastisch ist als die weitere Schicht. Der Stamm der Säule neigt daher weniger zur Agglomeration mit oder ohne Belastung. Gleichzeitig ist der obere Teil des Vorsprungs elastischer und kann sich besser auch an raue und/oder weiche Oberflächen anpassen.

In einer weiteren Ausführungsform der Erfindung ist die Grenzfläche zwischen weiterer Schicht und Stirnfläche nicht parallel zur Oberfläche der weiteren Schicht bezogen auf den jeweiligen Vorsprung.

In einer Ausführungsform der Erfindung ist die Stirnfläche eines Vorsprungs gekrümmt. Bevorzugt umfasst sie ein Maximum innerhalb des Vorsprungs. Insbesondere ist diese parabolisch oder halbkugelförmig geformt. Dadurch ist auch die Grenzfläche zur weiteren Schicht entsprechend geformt. Die Stirnfläche kann dabei auch nur zum Rand des Vorsprungs hin eine Krümmung aufweisen, während sie in der Mitte des Vorsprungs eben verläuft. Bei einer solchen Anordnung ist die Dicke der weiteren Schicht oberhalb der Stirnfläche nicht konstant. Bildet die Stirnfläche ein Maximum in der Mitte des Vorsprungs und die Oberfläche der weiteren Schicht ist plan geformt, so nimmt die Dicke der weiteren Schicht oberhalb des Vorsprungs zur Mitte des Vorsprungs hin ab.

Eine solche Form der Grenzfläche führt dazu, dass in diesem Bereich Materialien mit unterschiedlicher Elastizität oder Biegesteifigkeit vorliegen und ineinandergreifen. Es zeigte sich, dass eine solche Anordnung die Adhäsionskraft eines solchen Vorsprungs erhöht und auch die Neigung zum Kollabieren verringert.

In einer Ausführungsform der Erfindung beträgt das Verhältnis der minimalen senkrechte Dicke der weiteren Schicht oberhalb des Vorsprungs im Verhältnis zur Höhe des Vorsprungs weniger als 3, bevorzugt weniger als 1, insbesondere weniger als 0,5, insbesondere weniger als 0,2. Dadurch wirken sich Veränderungen der Dicke der weiteren Schicht, z. B. bei gekrümmten Phasengrenzflächen durch die Geometrie der Vorsprünge, besonders stark auf die Adhäsion aus. Das optimale Verhältnis kann auch vom Verhältnis der Elastizitätsmodule, sowie der Geometrie der Grenzfläche abhängen.

In einer Ausführungsform der Erfindung ist die Krümmung der Stirnfläche konvex in Richtung der weiteren Schicht, d.h. die Phasengrenzfläche weist ein Maximum auf. Bevorzugt ist die Krümmung eine kugelförmige Krümmung, insbesondere mit einem Radius bis zu dem doppelten Durchmesser des Vorsprungs, insbesondere mindestens dem Durchmesser des Vorsprungs.

In einer weiteren Ausführungsform der Erfindung weist der Vorsprung bei Ablösung von einer Oberfläche einen Beginn der Ablösung in der Mitte des Vorsprungs auf. Die vorteilhaften Parameter für Elastizitätsmodul, Größenverhältnis und Geometrie der Grenzfläche, insbesondere konvexe Grenzfläche, können durch Simulationen und Messungen bestimmt werden.

In einer bevorzugten Ausführungsform der Erfindung sind die Vorsprünge auf der Trägerschicht säulenartig ausgebildet. Dies bedeutet, dass es sich um bevorzugt senkrecht zur Trägerschicht ausgebildete Vorsprünge handelt, welche einen Stamm und eine Stirnfläche aufweisen, wobei der Stamm und die Stirnfläche einen beliebigen Querschnitt aufweisen können (beispielsweise kreisförmig, oval, rechteckig, quadratisch, rautenförmig, sechseckig, fünfeckig, etc.).

Bevorzugt sind die Vorsprünge so ausgebildet, dass die senkrechte Projektion der Stirnfläche auf die Grundfläche des Vorsprungs mit der Grundfläche eine Überlappungsfläche bildet, wobei die Überlappungsfläche und die Projektion der Überlappungsfläche auf die Stirnfläche einen Körper aufspannt, welcher vollständig innerhalb des Vorsprungs liegt. In einer bevorzugten Ausführungsform der Erfindung umfasst die Überlappungsfläche mindestens 50 % der Grundfläche, bevorzugt mindestens 70 % der Grundfläche, besonders bevorzugt umfasst die Überlappungsfläche die gesamte Grundfläche. Die Vorsprünge sind daher bevorzugt nicht geneigt, können es aber sein.

In einer bevorzugten Ausführungsform ist die Stirnfläche parallel zur Grundfläche und zur Oberfläche ausgerichtet. Falls die Stirnflächen nicht parallel zur Oberfläche ausgerichtet sind und daher verschiedene senkrechte Höhen aufweisen, wird als senkrechte Höhe des Vorsprungs die mittlere senkrechte Höhe der Stirnfläche angesehen.

In einer bevorzugten Ausführungsform der Erfindung weist der Stamm des Vorsprungs bezogen auf seinen mittleren Durchmesser ein Aspektverhältnis von Höhe zu Durchmesser von 1 bis 100, bevorzugt von 1 bis 10, besonders bevorzugt von 2 bis 5 auf.

In einer Ausführungsform liegt das Aspektverhältnis bei größer 1, bevorzugt bei mindestens 3, insbesondere bei mindestens 7, bevorzugt bei 3 bis 15, besonders bevorzugt bei 3 bis 10.

Unter dem mittleren Durchmesser wird dabei der Durchmesser des Kreises verstanden, der die gleiche Fläche wie der entsprechende Querschnitt des Vorsprungs aufweist, gemittelt über die gesamte Höhe des Vorsprungs.

In einer weiteren Ausführungsform der Erfindung liegt das Verhältnis der Höhe eines Vorsprungs zum Durchmesser bei einer bestimmten Höhe über die gesamte Höhe des Vorsprungs immer bei 1 bis 100, bevorzugt bei 1 bis 10, besonders bevorzugt bei 2 bis 5. In einer Ausführungsform liegt dieses Aspektverhältnis bei mindestens 3, insbesondere bei mindestens 7, bevorzugt bei 3 bis 15, besonders bevorzugt bei 3 bis 10. Dabei wird unter Durchmesser der Durchmesser des Kreises verstanden, der die gleiche Fläche wie der entsprechende Querschnitt des Vorsprungs bei der bestimmten Höhe aufweist.

Die Vorsprünge können verbreiterte Stirnflächen aufweisen, sogenannte "mushroom"-Strukturen. Es ist auch möglich, dass die weitere Schicht über die Stirnfläche hinausragt und so eine "mushroom"-Struktur bildet.

In einer bevorzugten Ausführungsform weisen die Vorsprünge keine verbreiterten Stirnflächen auf.

Die Oberfläche der weiteren Schicht kann selbst strukturiert sein, um ihre Oberfläche zu erhöhen. In diesem Fall wird als senkrechte Dicke der weiteren Schicht die mittlere senkrechte Höhe der weiteren Schicht angesehen.

In einer bevorzugten Ausführungsform liegt die senkrechte Höhe aller Vorsprünge in einem Bereich von 1 µm bis 10 mm, bevorzugt 1 µm bis 5 mm, insbesondere 1 µm bis 2 mm, bevorzugt in einem Bereich von 10 µm bis 2 mm.

In einer bevorzugten Ausführungsform liegt die senkrechte Dicke der weiteren Schicht oberhalb einer Stirnfläche in einem Bereich von 1 µm bis 1 mm, bevorzugt 1 µm bis 500 µm, insbesondere 1 µm bis 300 µm, bevorzugt in einem Bereich von 1 µm bis 200 µm, insbesondere in einem Bereich von 10 µm bis 200 µm, ganz besonders 5 µm bis 100 µm.

Bevorzugt weist die weitere Schicht bezogen auf mind. 50 % der Projektion der Grundfläche eines Vorsprungs auf die Oberfläche der weiteren Schicht eine senkrechte Dicke in dem vorstehenden Bereich oder einem der bevorzugten Bereiche auf.

Die kleinste Dicke der weiteren Schicht oberhalb eines Vorsprungs ist bevorzugt immer kleiner als die maximale senkrechte Höhe des Vorsprungs.

In einer bevorzugten Ausführungsform liegt die senkrechte Dicke der Trägerschicht in einem Bereich von 1 µm bis 2 mm, bevorzugt 1 µm bis 500 µm, insbesondere 1 µm bis 300 µm.

In einer bevorzugten Ausführungsform entspricht die Grundfläche von der Fläche her einem Kreis mit einem Durchmesser zwischen 0,1 µm bis 5 mm, bevorzugt 0,1 µm und 2 mm, insbesondere bevorzugt zwischen 1 µm und 500 µm, besonders bevorzugt zwischen 1 µm und 100 µm. In einer Ausführungsform ist die Grundfläche ein Kreis mit einem Durchmesser zwischen 0,3 µm und 2 mm, bevorzugt 1 µm und 100 µm.

Der mittlere Durchmesser der Stämme liegt bevorzugt zwischen 0,1 µm bis 5 mm, bevorzugt 0,1 µm und 2 mm, insbesondere bevorzugt zwischen 1 µm und 100 µm. Bevorzugt ist die Höhe und der mittlere Durchmesser entsprechend dem bevorzugten Aspektverhältnis angepasst.

In einer bevorzugten Ausführungsform ist bei verbreiterten Stirnflächen die Oberfläche der Stirnfläche eines Vorsprungs, bzw. die Oberfläche der weiteren Schicht, mindestens 1,01 mal, bevorzugt mindestens 1,5 mal so groß wie die Fläche der Grundfläche eines Vorsprungs. Sie kann beispielsweise um den Faktor 1,01 bis 20 größer sein.

In einer weiteren Ausführungsform ist die verbreiterte Stirnfläche zwischen 5% und 100% größer als die Grundfläche, besonders bevorzugt zwischen 10% und 50% der Grundfläche.

In einer bevorzugten Ausführungsform beträgt der Abstand zwischen zwei Vorsprüngen weniger als 2 mm, insbesondere weniger als 1 mm, ganz besonders weniger als 500 µm oder weniger als 100 µm.

Die Vorsprünge sind bevorzugt regelmäßig periodisch angeordnet.

In einer bevorzugten Ausführungsform der Erfindung weisen die Vorsprünge eine Höhe von 5 bis zu 50 µm, bevorzugt bis zu 25 µm auf. Die weitere Schicht weist oberhalb der Stirnflächen eine senkrechte Dicke von 3 bis 70 µm. Der mittlere Abstand der säulenförmigen Vorsprünge liegt zwischen 5 und 50 µm. Die Dicke der Trägerschicht liegt zwischen 5 und 100 µm. Der Durchmesser liegt abhängig vom Abstand der Vorsprünge bei 5 bis 40 µm. Bevorzugt sind die Vorsprünge hexagonal angeordnet. Besonders bevorzugt liegt die Dichte der Vorsprünge bei 10000 bis 1000000 Vorsprüngen/cm².

Die gesamte Dicke der Vorrichtung umfassend weitere Schicht, Vorsprünge und Trägerschicht liegt bevorzugt zwischen 50 µm und 300 µm.

Die Elastizitätsmodule aller Bereiche des Vorsprungs und der weiteren Schicht liegen bevorzugt bei 50 kPa bis 3 GPa. Bevorzugt liegt der Elastizitätsmodul von weichen Bereichen, d. h. insbesondere der weiteren Schicht, bei 50 kPa bis 20 MPa, bevorzugt 100 kPa bis 10 MPa. Bevorzugt liegt davon unabhängig der Elastizitätsmodul der Bereiche mit hohem Elastizitätsmodul, z. B. der Vorsprünge sowie z. B. der Trägerschicht, bei 1 MPa bis 3 GPa, bevorzugt 2 MPa bis 1 GPa. Bevorzugt liegen für alle weicheren und härteren Bereiche die Elastizitätsmodule in den vorstehend angegebenen Bereichen.

Das Verhältnis der Elastizitätsmodule zwischen dem geringsten Elastizitätsmodul und dem Bereich mit dem höchsten Elastizitätsmodul liegt bevorzugt bei unter 1:2000, insbesondere bei unter 1:1500, bevorzugt bei unter 1:1200, davon unabhängig mindestens bei 1:1,1, bevorzugt mindestens 1:1,5, insbesondere bei mindestens 1:2. Dabei kann ein Verhältnis von bis zu 1:1000 vorteilhaft sein. Bevorzugt weist die weitere Schicht den geringsten Elastizitätsmodul auf. Insbesondere liegt das Verhältnis bei 1:1,1 bis 1:500, bevorzugt bei 1:2 bis 1:500.

In einer weiteren Ausführungsform der Erfindung liegt das Verhältnis der Elastizitätsmodule des Bereichs der Vorrichtung mit dem geringsten Elastizitätsmodul und dem Bereich mit dem höchsten Elastizitätsmodul bevorzugt bei 1:2 bis 1:200 (weich zu hart), insbesondere bei 1:2 bis 1:100.

In einer weiteren Ausführungsform beschreiben die vorstehend angegebenen Verhältnisse das Verhältnis der Elastizitätsmodule der weiteren Schicht (weich) und der Vorsprünge (hart).

In einer besonders bevorzugten Ausführungsform der Erfindung füllt die weitere Schicht zusätzlich die Zwischenräume zwischen den Vorsprüngen aus.

Bei dieser Ausführungsform kann die Vorrichtung als Beschichtung aus mindestens zwei Komponenten gesehen werden, wobei diese beiden Komponenten aufgrund der nun in die Beschichtung eingebetteten Vorsprünge zueinander eine strukturierte Grenzfläche aufweisen. Die Oberseite der weiteren Schicht ist bevorzugt planar geformt, ebenso bevorzugt auch die Unterseite der Trägerschicht. Die Angaben zur Dicke der weiteren Schicht beziehen sich für den oberhalb der Stirnflächen angeordneten Teil der weiteren Schicht.

Durch das Auffüllen der Zwischenräume werden die Vorsprünge zusätzlich stabilisiert. Es war überraschend zu finden, dass auch solche Schichten eine erhöhte Adhäsion aufweisen. Auch werden die eingeschlossenen Vorsprünge durch das sie umgebende Material stabilisiert. Dadurch führen auch Zugkräfte parallel zur Kontaktfläche der Vorrichtung nicht zum Kollabieren der Vorsprünge, sondern die Adhäsionskraft bleibt auch bei solchen Zugkräften vorhanden. Dies ist beispielsweise wichtig, wenn die Vorrichtung neben der Adhäsion auch Zugkräfte parallel zur Kontaktfläche aushalten soll. Beispielweise beim Aufbringen auf zu schließende Wunden oder Verletzungen von Trommelfellen.

Außerdem ist diese Schicht einfach sauber, bzw. steril, zu halten, da sich keinerlei Verschmutzung in den Zwischenräumen ansammeln kann.

Durch die gefüllte Struktur können zusätzlich Spannungsspitzen, welche bei freistehenden Vorsprüngen beim Ablösen von einer Oberfläche auftreten, reduziert werden.

Zusätzlich kann eine solche Vorrichtung einfacher hergestellt werden, da die Trägerschicht mit den Vorsprüngen lediglich mit dem Material der weiteren Schicht überschichtet werden muss. Aufwendige Strukturierungsschritte sind nicht erforderlich.

Dadurch erscheint die Oberfläche der Vorrichtung in dieser Ausführungsform geschlossen und einheitlich. Dadurch kann sie auch einfacher modifiziert werden, um für Anwendungen angepasst zu werden. Eine Behandlung der Oberfläche wirkt sich dann nicht auf die Strukturierung innerhalb der Beschichtung aus.

So kann die Oberfläche mit bekannten Verfahren funktionalisiert oder behandelt werden.

In einer weiteren Ausführungsform der Erfindung ist die weitere Schicht Teil eines Films, welcher die Vorsprünge verbindet. In diesem Fall werden die Zwischenräume zwischen den Vorsprüngen nicht ausgefüllt.

Der Film weist bevorzugt eine Dicke auf, welche unter 100%, bevorzugt unter 50 %, besonders bevorzugt unter 30 % der senkrechten Höhe der überbrückten Vorsprünge liegt. Dabei wird der Film nicht in die Berechnung der Höhe einbezogen.

Der Film weist bevorzugt eine Dicke von unter 2 mm, bevorzugt von unter 1 mm, besonders bevorzugt von unter 800 µm auf.

Die Vorsprünge können aus vielen unterschiedlichen Materialien bestehen, bevorzugt sind Elastomere, besonders bevorzugt vernetzbare Elastomere. Die Bereiche mit höherem Elastizitätsmodul können auch Duroplaste umfassen.

Die Vorsprünge sowie die weitere Schicht können daher folgende Materialien umfassen:
epoxy- und/oder silikonbasierte Elastomere, Polyurethane, Epoxidharze, Acrylatsysteme, Methacrylatsysteme, Polyacrylate als Homo- und Copolymere, Polymethacrylate als Homo- und Copolymere (PMMA, AMMA Acrylnitril/Methylmethacrylat), Polyurethan(meth)acrylate, Silikone, Silikonharze, Kautschuk, wie R-Kautschuk (NR Naturkautschuk, IR Poly-Isopren-Kautschuk, BR Butadienkautschuk, SBR Styrol-Butadien-Kautschuk, CR Chloropropen-Kautschuk, NBR Nitril-Kautschuk, M-Kautschuk (EPM Ethen-Propen-Kautschuk, EPDM Ethylen-Propylen-Kautschuk), Ungesättigte Polyesterharze, Formaldehydharze, Vinylesterharze, Polyethylene als Homo- oder Copolymere, sowie Mischung und Copolymere der vorgenannten Materialien. Bevorzugt sind auch Elastomere, welche zur Verwendung im Bereich Verpackung, Pharma und Lebensmittel von der EU (gemäß EU-VO Nr. 10/2011 vom 14.01.2011, veröffentlicht am 15.01.2011) oder FDA zugelassen sind oder silikonfreie UV-härtbare Harze aus der PVD und CVD-Verfahrenstechnik. Dabei steht Polyurethan(meth)acrylate für Polyurethanmethacrylate, Polyurethanacrylate, sowie Mischungen und/oder Copolymere davon.

Es kann sich auch um Hydrogele, beispielsweise auf Basis von Polyurethanen, Polyvinylpyrrolidon, Polyethylenoxid, Poly(2-acrylamido-2-methyl-1-propanesulfonsäure, Silikonen, Polyacrylamiden, hydroxylierten Polymethacrylaten oder Stärke, handeln.

Bevorzugt sind epoxy- und/oder silikonbasierte Elastomere, Polyurethan(meth)acrylate, Polyurethane, Silikone, Silikonharze (wie UV-härtbares PDMS), Polyurethan(meth)acrylate, Kautschuk (wie EPM, EPDM).

Besonders bevorzugt sind vernetzbare Silikone wie beispielweise Polymere auf Basis Vinyl-terminierter Silikone.

Insbesondere für die weitere Schicht, welche in Kontakt mit der Oberfläche steht, sind von den vorstehend genannten die epoxy- und/oder silikonbasierte Elastomere, Polyurethan(meth)acrylate, Polyurethane, Silikone, Silikonharze (wie UV-härtbares PDMS), Polyurethan(meth)acrylate, Kautschuk (wie EPM, EPDM), insbesondere vernetzbare Silikone wie beispielweise Polymere auf Basis Vinyl-terminierter Silikone, bevorzugt.

Es können auch die vorstehend genannten Hydrogele oder druckempfindliche Klebstoffe für die weitere Schicht verwendet werden.

In einer weiteren Ausführungsform wird die Oberfläche der weiteren Schicht behandelt. Dadurch können die Eigenschaften der Oberfläche beeinflusst werden. Dies kann durch physikalische Behandlung wie Plasmabehandlung, bevorzugt mit Ar/O₂-Plasma, geschehen.

Es können auch kovalente oder nicht kovalente Bindungen zu Additiven auf der Oberfläche ausgebildet werden, beispielsweise um eine bestimmte Kompatibilität mit den Zellen zu erreichen. Bevorzugt sind Additive zur Unterstützung der Zelladhäsion, wie z. B. Poly-L-Lysin, Poly-L-Ornithin, Collagen oder Fibronectin. Solche Additive sind aus dem Bereich der Zellkultur bekannt.

Gerade bei Anwendung im medizinischen Bereich kann es auch vorteilhaft sein, Stoffe in mindestens einen Teil der Vorrichtung einzulagern, welche dann langsam abgegeben werden. Dies können beispielsweise Arzneimittel, wie Antibiotika sein, oder auch Hilfsstoffe zur Unterstützung der Zelladhäsion oder des Zellwachstums.

In einer weiteren Ausführungsform sind die Vorsprünge und die Trägerschicht einstückig ausgeführt und bestehen aus dem gleichen Material.

In einer weiteren Ausführungsform umfasst die Vorrichtung noch weitere Schichten, welche gegebenenfalls ablösbar sind. So können die Oberflächen durch ablösbare Folien vor dem Einsatz geschützt werden. Auch können weitere stabilisierende Schichten auf der Trägerschicht angeordnet sein.

Die Trägerschicht weist bevorzugt eine geringere Dicke als die maximale Höhe der auf ihr angeordneten Vorsprünge auf.

Da die Trägerschicht, wenn sie aus dem gleichen Material wie die Vorsprünge besteht, ein Material mit einem höheren Elastizitätsmodul umfasst, kann mit der Dicke der Trägerschicht auch die Elastizität der gesamten Vorrichtung beeinflusst werden. Die erfindungsgemäße Vorrichtung ist bevorzugt zur Adhäsion auf weichen Substraten ausgebildet.

Die erfindungsgemäße Vorrichtung ist insbesondere zur Adhäsion auf biologischen Geweben ausgebildet. Dazu kann sie beispielsweise als Folie ausgeführt sein. Sie kann auch in Kombination mit den zu befestigenden Vorrichtungen ausgeführt sein. Dies können beispielsweise Verbandsmaterial sein aber auch Elektroden oder andere medizinische Vorrichtungen wie Implantate, insbesondere Implantate, welche nicht an Knochen verankert werden sollen, oder weiche Implantate. Dies können beispielsweise Irisimplantate sein. Die Erfindung betrifft daher auch ein Implantat umfassend eine erfindungsgemäße Vorrichtung, beispielsweise auf mindestens einem Teil der Oberfläche des Implantats.

### Behandlung von Trommelfellperforationen

Durch die Adhäsion der Vorrichtung haftet die Vorrichtung sehr gut an der Oberfläche des Trommelfells und erlaubt es sogar, unter Spannung aufgebracht zu werden. Aufgrund ihrer Struktur haftet sie auch auf den umgebenden Geweben und nicht nur auf dem Trommelfell. Gegebenenfalls kann die derart ausgebildete Vorrichtung unterschiedliche Bereiche mit unterschiedlicher Adhäsion umfassen. Dies kann beispielsweise über das Material, die Schichtdicke der weiteren Schicht, aber auch einfach durch die Verteilung der Vorsprünge innerhalb der Vorrichtung erfolgen.

Die vorteilhafterweise als Folie ausgeführte Vorrichtung umfasst daher mindestens die Trägerschicht mit den Vorsprüngen und auf diese Vorsprünge ist die weitere Schicht so aufgebracht, dass die Zwischenräume ausgefüllt sind. Durch die Ausführung als Folie kann die Vorrichtung auf einfache Weise auf die gewünschte Größe zugeschnitten werden. Dies kann sogar durch die behandelnde Person, z. B. dem Arzt, selber erfolgen.

Durch ihre innere Strukturierung haftet die Vorrichtung gut an dem Gewebe, auf das sie aufgebracht wird. Dies kann neben dem Trommelfell auch das umgebende Gewebe sein. Es wird kein flüssiger Bestandteil zum Aufbringen der Vorrichtung benötigt, welcher in das Ohr hineinfließen kann.

Die Vorrichtung kann abhängig von den verwendeten Materialien auch transparent sein, so dass ohne Ablösen der Zustand des Gewebes unterhalb der Vorrichtung untersucht werden kann, beispielsweise um die Verheilung festzustellen. Bevorzugt ist die Vorrichtung transparent.

Durch die Trockenadhäsivität kann die Vorrichtung einfach wieder abgelöst werden.

schnitten werden. Über den E-Modul der beiden Schichten, insbesondere der weiteren Schicht, auf welcher die Zellen kultiviert werden, kann die Vorrichtung auf die zu kultivierenden Zellen angepasst werden (E-Modul(Gehirnzellen): ca. 0,1-1 kPa; E-Modul (Muskelzellen): ca. 8-17 kPa; E-Modul(Knochenzellen): ca. 25-40 kPa).

Die Vorrichtung kann insbesondere nach der Kultivierung der Zellen auf einfache Weise geteilt werden, so dass verschiedene Untersuchungen ausgehend von der gleichen Zellkultur durchgeführt werden können. Da die Vorrichtung transparent sein kann, sind die so erhaltenen Kulturen auch für mikroskopische Untersuchungen geeignet. Sie können beispielsweise mittels Rasierklinge oder Skalpell geschnitten werden. Die Vorrichtung kann auch zur Kultivierung der Zellen auf übliche Glasträger, z. B. Rundgläschen, aufgebracht werden. Alternativ kann die Vorrichtung auch direkt zur Kultivierung in entsprechenden Kultivierbehältnissen verwendet werden.

Zur Kultivierung kann die Oberfläche der Vorrichtung auch physikalisch oder chemisch behandelt werden. Dies kann beispielsweise eine Autoklavierung sein, beispielsweise durch Dampfsterilisation bei 50 bis 200 °C, insbesondere 100 bis 150 °C, bei einem Druck von 1 bis 5 bar für 5 Minuten bis 3 Stunden. Bei einer solchen Autoklavierung (121 °C, 2 bar, 20 Minuten) konnte keine signifikante Veränderung der Haftspannung beobachtet werden.

So kann die Oberfläche beispielsweise mit Poly-L-Lysin, Poly-L-Ornithin, Collagen, Fibronectin, Gelatine, Laminine, Keratin, Tenascin oder Perlecan behandelt werden. Solche Additive sind aus dem Bereich der Zellkultur bekannt.

Die Erfindung betrifft außerdem ein Verfahren zur Herstellung einer Ausführungsform der erfindungsgemäßen Vorrichtung.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Dazu wird in einem ersten Schritt ein Templat zur Abformung der Vielzahl an Vorsprüngen bereitgestellt.

In dieses Templat wird das Material für die Vorsprünge eingebracht, bevorzugt als Flüssigkeit. Gegebenenfalls kann das Material auch bereits mindestens teilweise gehärtet werden.

Danach wird das Material für die Trägerschicht, d.h. die Oberfläche, auf der die Vorsprünge angeordnet sind, auf das Templat aufgebracht und gehärtet. Besonders bevorzugt ist dies das gleiche Material wie für die Stämme der Vorsprünge, so die Trägerschicht und die Stämme auch in einem Schritt hergestellt werden, beispielsweise, indem direkt eine größere Menge an Material eingebracht wird.

In einem nächsten Schritt werden die Trägerschicht und die Vorsprünge aus dem Templat gelöst.

Danach wird das Material für die weitere Schicht in der Menge auf die Oberfläche mit den Vorsprüngen aufgebracht, so dass die Vorsprünge vollständig bedeckt werden. Dies kann beispielsweise durch Rakeln oder Spin coating erfolgen.

In einem nächsten Schritt wird die weitere Schicht gehärtet.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
- Fig. 1: Schematische Darstellung eines Querschnitts einer Ausführungsform der Erfindung;
- Fig. 2: REM-Aufnahmen von erfindungsgemäßen Schichten; Hergestellt durch Spin coating von SSA 50:50 auf eine mikrostrukturierte PDMS-Schicht bei 6000 rpm; Die PDMS-Schicht wurde mit Gewichten (100g) hergestellt;
- Fig. 3: Abhängigkeit der Schichtdicke über der mikrostrukturierten PDMS-Schicht von der Geschwindigkeit beim Spin coating; Alle PDMS-Schichten wurden mit Gewichten (100 g) hergestellt;
- Fig. 4: Statistische Auswertung der erhaltenen Schichtdicke in Abhängigkeit von der Verwendung von Gewichten;
- Fig. 5: REM-Aufnahme eines Querschnitts durch eine mittels Spin coating aufgetragene Schicht von SSA 50:50 auf ein Glassubstrat;
- Fig. 6: Abhängigkeit der Schichtdicke von SSA 50:50 auf einer Glasoberfläche von der Geschwindigkeit beim Spin coating;
- Fig. 7: Adhäsionskraft von SSA und PDMS in unterschiedlichen Verhältnissen gegen ein glattes Substrat (SMOOTH) in verschiedenen Auftragungen; (A: Abzugsspannung (pulloff stress) gegen Geschwindigkeit (velocity); B: Abzugsspannung (pull-off stress) gegen Schichtdicke (thickness); C: maximale Dehnung (maximum strain) gegen Schichtdicke (thickness); D: Adhäsionsenergie (adhesion energy) gegen Schichtdicke (thickness)); die Schichtdicke wurde mit REM bestimmt;
- Fig. 8: Mikroskopische Aufnahmen von L929-Zellen 4 h nach Ausplattieren auf PDMS (A, C, E, G) oder SSA 50:50 (B, D, F); unmodifiziert (A und B); Modifiziert mit Poly-L-Lysin (C und D); Poly-L-Ornithin und anschließender Inkubation mit Fibronektin (E und F); G Zellen kultiviert auf Zellkulturbehandeltem (TC treated) Polysterol
- Fig. 9: Mikroskopische Aufnahmen von L929-Zellen nach 24 h auf PDMS (A), SSA 50:50 (B), sowie jeweils mit O₂/Ar-Plasma behandeltem PDMS (C) bzw. SSA 50:50 (D);
- Fig. 10: Zellzahl (cell number) nach 24 h auf den Oberflächen gemäß Figur 9 ausgehend von 3x10⁵ vitalen Zellen (student's t-Test, * p<0,05 ** p<0,0005);
- Fig. 11: Aktivität (in percent cytotoxicity) von Lactatdehydrogenase (LDH) nach 24 h Kultivierung auf den Oberflächen gemäß Fig. 9 und einer Kontrolle;
- Fig. 12: Vergleich der Adhäsionskraft von strukturierten Beschichtungen (PDMS/SSA 50:50; 20 µm Schichtdicke SSA 50:50 über den Vorsprüngen) und unstrukturierter Beschichtung PDMS/SSA 50:50;
- Fig. 13: Zugkraftversuche mit unstrukturierten Beschichtungen mit Vitro-Skin® (Aufbau gezeigt in A); B zeigt die gemessenen Zugkräfte für PDMS (Referenz) und PDMS, auf die SSA in unterschiedlichen Mischungsverhältnissen von 40:60 bis 52:48 aufgetragen wurde;
- Fig. 14: Mikroskopische Aufnahmen von L929-Zellen nach 48 h auf PDMS (A), SSA 50:50 (B), PDMS mit PLL (C), SSA 50:50 mit PLL (D); Aufnahmen A1, B1, C1 und D1 zeigen die Proben nach Schütteln für 60 s;
- Fig. 15: Kontaktwinkel für unterschiedliche Oberflächen vor und nach Plasmabehandlung (links nach rechts: PDMS; PDMS Plasma; SSA 40:60; SSA 40:60 Plasma; SSA 50:50; SSA 50:50 Plasma);
- Fig. 16: Das komplexe Modul (complex modulus) von SSA 40:60 und SSA 50:50 wurde mittels Rheometrie mit Frequenzen (Frequency) zwischen 0,1 und 100 Hz ermittelt. Die Messamplitude betrug 0,1%;

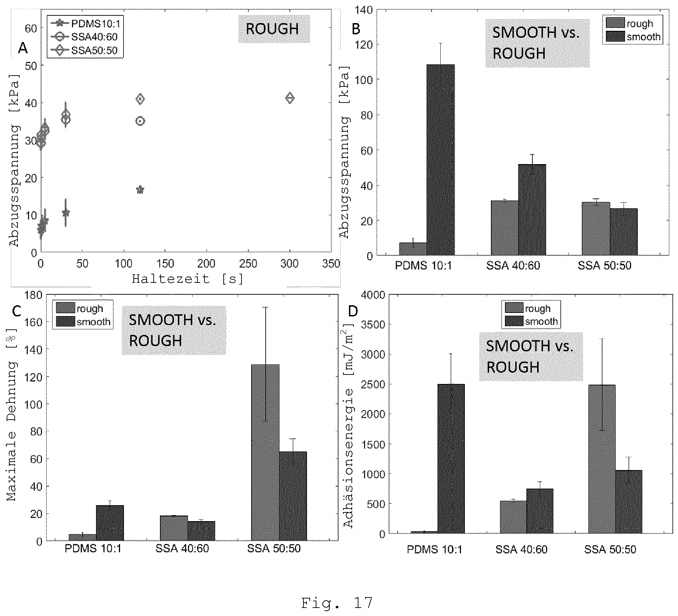

- Fig. 17: Vergleich verschiedener Adhäsionsparameter zwischen rauem (ROUGH) Substrat und glattem (smooth) Substrat: (A) Ermittlung der Abzugsspannung (pull-off stress) in Abhängigkeit der Verweildauer (holding time) nachdem das Substrat mit einer Kraft von etwa 40mN auf die Präparate gepresst wurde. Die Dicke der Präparate, die mittels rauem Substrat untersucht wurden, betrug zwischen 130 µm und 170 µm. (B) Vergleich der Abzugsspannung (pull-off stress) bei Verwendung eines rauen Substrats und eines glatten Substrats. (C) Ermittlung der maximalen Dehnung (maximum strain) bei Verwendung eines rauen Substrats und eines glatten Substrats. (D) Ermittlung der Adhäsionsenergie (adhesion energy) bei Verwendung eines rauen Substrats und eines glatten Substrats.
- Fig. 18: Schematischer Aufbau der Messapparatur, die zur Ermittlung der Adhäsionswerte verwendet wurde (A). Ermittlung der Rauigkeit der für die Messungen verwendeten Glasssubstrate. Die Kurve (Entfernung (distance) gegen Höhe (height) für das glatte Substrat (smooth) zeigt fast keine Ausschläge im Gegensatz zum rauen Substrat (rough).
- Fig. 19: Schematische Darstellung des Herstellungsprozesses;
- Fig. 20: Schematische Darstellung von Querschnitten von weiteren Ausführungsformen der Erfindung.

Für die Versuche wurde Soft Skin Adhesive (SSA) von Dow Corning verwendet. Dies sind Vinyl-terminierte Silikone. Durch Mischung zweier Lösungen A und B wird das Aushärten der Polymere katalysiert und Pt eingeleitet. Die Versuche wurden mit MG 7-9800 durchgeführt. Die verwendeten Zusammensetzungen werden in SSA A:B angegeben.

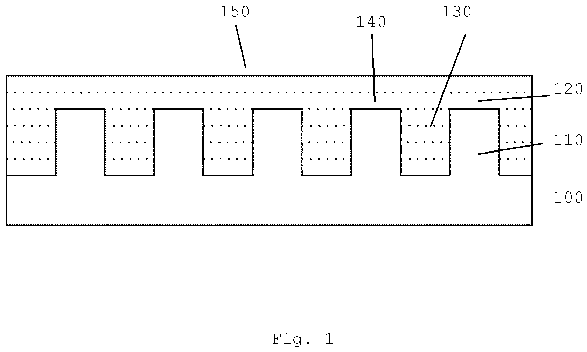

Figur 1 zeigt eine schematische Darstellung eines Querschnitts einer Ausführungsform der Erfindung. Die Vorrichtung umfasst eine Trägerschicht (100), auf der eine Vielzahl von Vorsprüngen (110) angeordnet sind. Auf der Stirnfläche (140) des jeweiligen Vorsprungs ist eine weitere Schicht (120) angeordnet. Dabei füllt diese Schicht auch die Zwischenräume (130) zwischen den Vorsprüngen. Die Oberfläche (150) der weiteren Schicht ist die Oberfläche, welche zur Adhäsion verwendet wird. Die Vorsprünge selbst sind bevorzugt kreisförmig im Querschnitt und stellen daher Säulen dar.

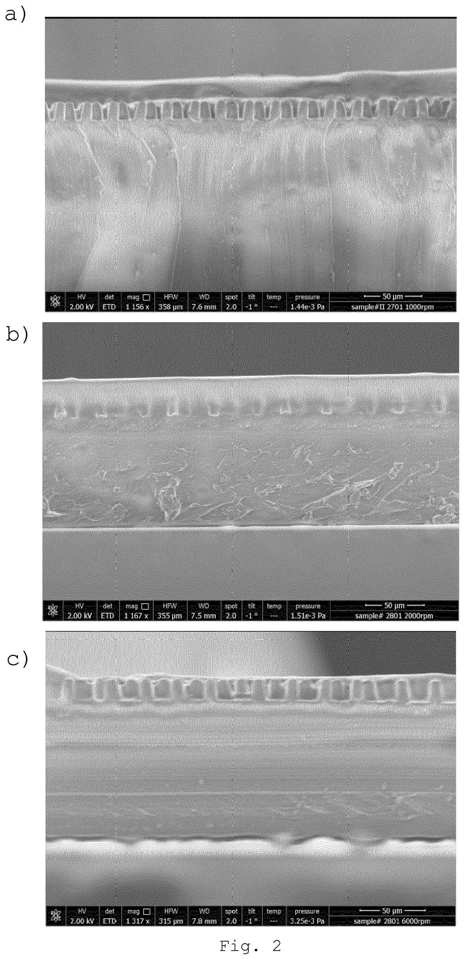

Figur 2 zeigt REM-Aufnahmen (REM: Rasterelektronenmikroskop), die den Einfluss der zunehmenden Zentrifugalbeschleunigung bei der Herstellung der SSA Schicht auf einer mikrostrukturierten PDMS Oberfläche zeigen. Deutlich ist die Abnahme der Schichtdicke mit zunehmender Drehzahl erkennbar.

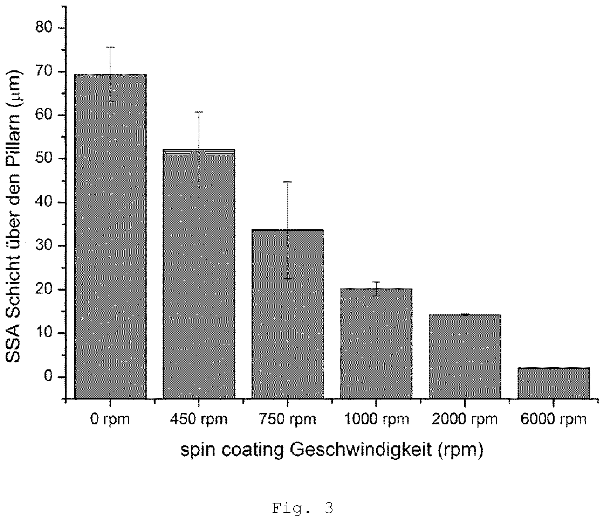

Figur 3 zeigt die mit Hilfe von REM bestimmte Schichtdicke bei unterschiedlichen Geschwindigkeiten (jeweils für 90 s). Als Schichtdicke gilt dabei die Dicke der Schicht oberhalb der mikrostrukturierten PDMS-Oberfläche.

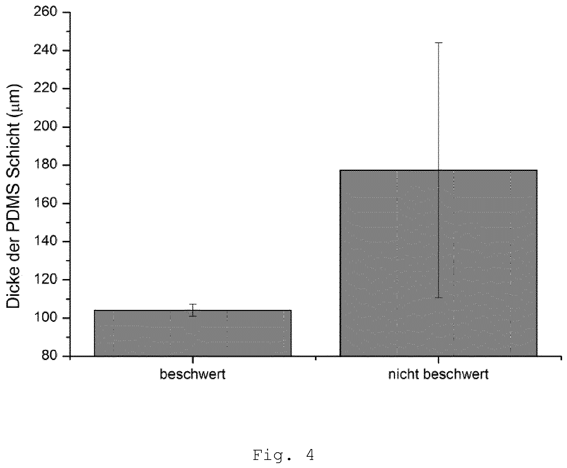

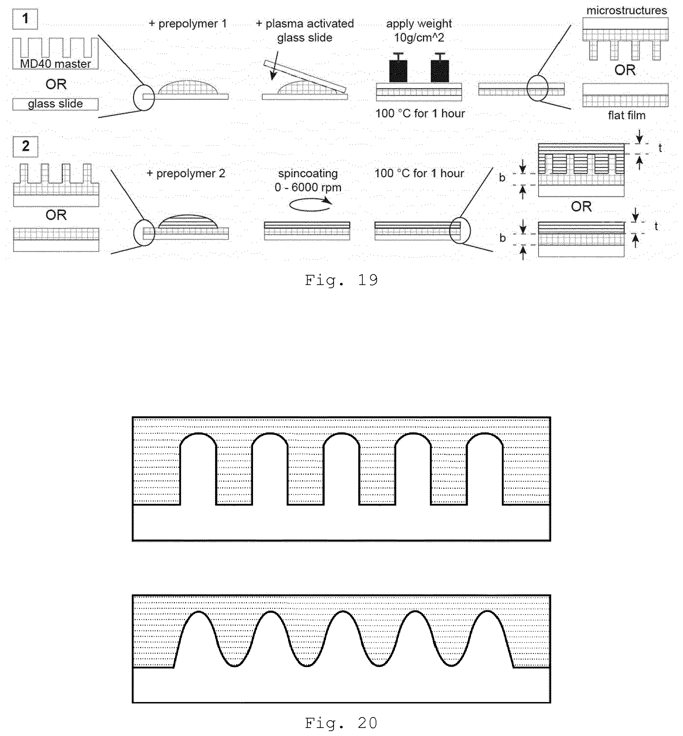

Figur 4 zeigt den Einfluss der Gewichte auf die Schichtdicke der hergestellten PDMS-Trägerschicht (siehe auch Figur 19 oberes Verfahren). Durch das Beschweren der Schicht können dünnere Schichten erhalten werden. Dadurch werden flexiblere Vorrichtungen erhalten.

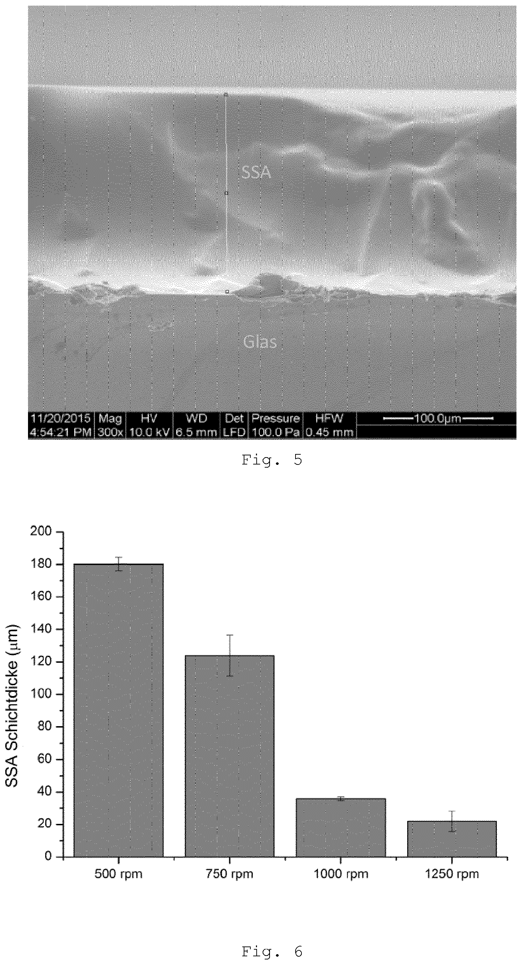

Figur 5 zeigt eine REM-Aufnahme einer Schicht SSA 50:50 auf Glas. Auch dort kann die Schichtdicke mittels den Bedingungen beim Spin coating eingestellt werden. Die entsprechend erhaltenen Schichtdicken sind in Figur 6 dargestellt. Die Zeit betrug immer 90 s. Ähnliche Schichten können aber auch bei geringerer Geschwindigkeit und längerer Dauer erhalten werden.

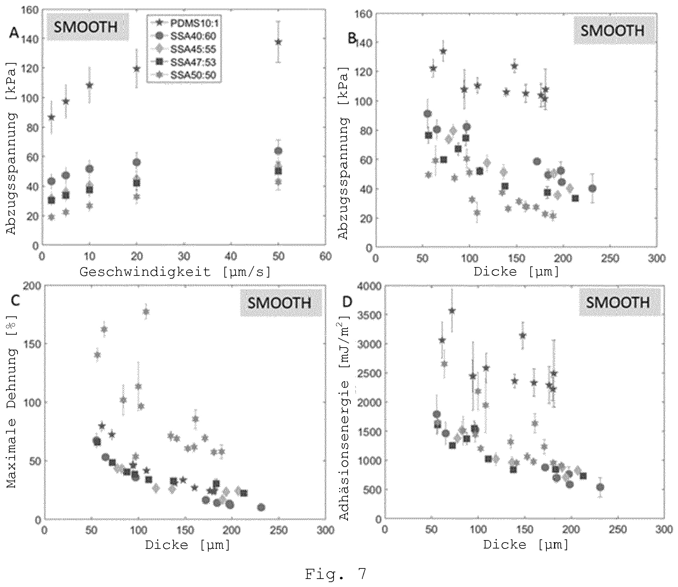

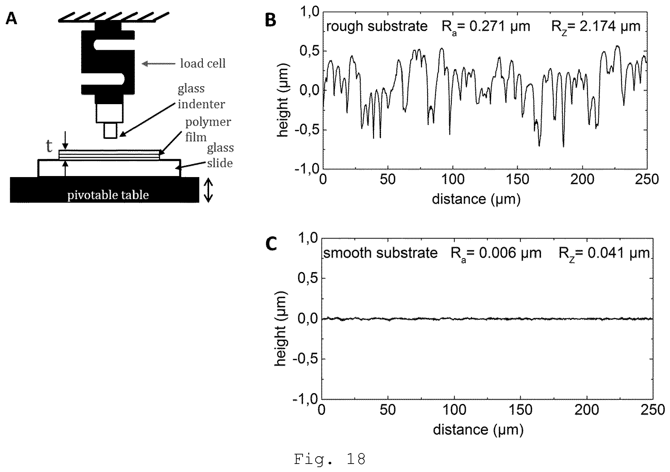

Figur 7 zeigt die gemessenen Werte einer Messung nach Figur 18 in verschiedenen Auftragungen. Es wurde PDMS (Slygard 184) verwendet. Das Verhältnis gibt die Anteile PDMS und Vernetzer an.

Unterschiedliche Schichtdicken (50 µm bis 250 µm) wurden mittels des Rakelverfahrens auf eine Glasoberfläche aufgetragen. Mit abnehmender Schichtdicke wurde eine Zunahme der Adhäsion gemessen. Bei allen Materialien lässt sich erkennen, dass eine Erhöhung der Abzugsgeschwindigkeit zu höheren Haftspannungen führt(Figur A). Für alle SSA Gemische gilt, dass eine ausgesprochene Abhängigkeit zwischen Filmdicke und allen untersuchten Parametern besteht. Dazu zählen Haftspannung (Figur B), maximale Dehnung (Figur C) und Adhäsionsenergie (Figur D). Aufgrund des erheblich größeren E-Moduls von PDMS findet sich dort eine wesentlich geringere Abhängigkeit der Haftspannung von der Filmdicke. Gerade aus Figur B lässt sich ableiten, dass die Haftspannung vom E-Modul der Materialien abhängt. Je steifer das Material ist, desto höhere Spannungen wurden beobachtet. Bei der Betrachtung der maximalen Dehnung (Figur C) fällt auf, daß die maximale Dehnung von SSA 50:50 deutlich größer ist, als alle anderen untersuchten Materialien.

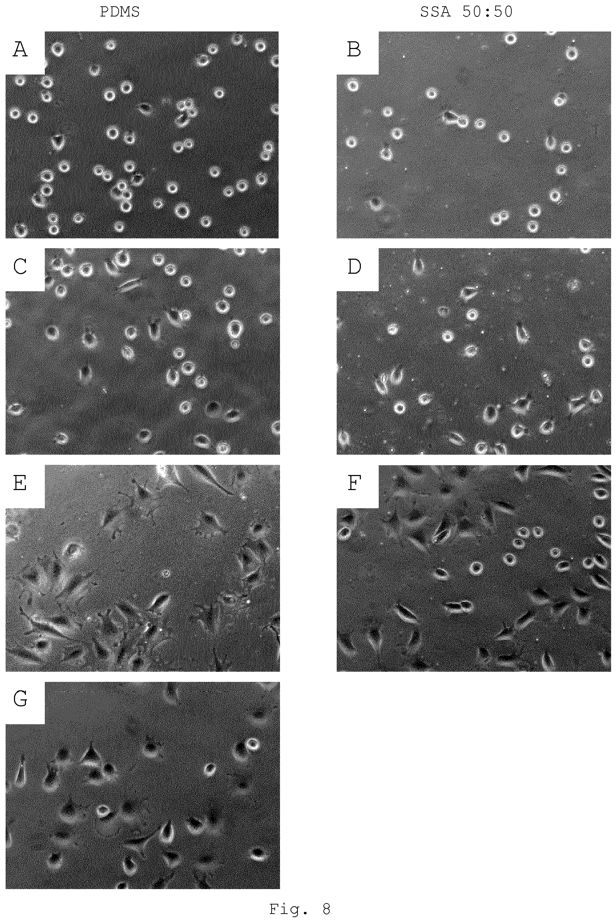

Figur 8 zeigt den Einfluss von Oberflächenmodifikation auf die Adhäsion von L929 Zellen (Fibroblasten, Spezies Maus). Dazu wurden solche Zellen nach einer Ausplattierungszeit von 4h auf den jeweiligen Oberflächen mikroskopisch untersucht. Bei PDMS und SSA 50:50 war eine minimale Zelladhäsion erkennbar, wenn die Oberflächen nicht modifiziert sind (A und B). Eine Adsorption von Poly-L-Lysin auf der Oberfläche führte zu einer deutlichen Zunahme des Adhäsionsverhaltens und der Ausbildung von zellulären Fortsätzen für PDMS (C) und SSA 50:50 (D).

Dieses Adhäsionsverhalten konnte durch eine Behandlung der Polymeroberfläche mit Poly-L-Ornithin und anschließender Inkubation mit Fibronectin für PDMS (E) und SSA 50:50 (F) deutlich verbessert werden. Die abgeflachte zelluläre Morphologie ist dabei vergleichbar mit zellkulturbehandeltem Polystyrol. Die Klebeeigenschaften des SSA 50:50 blieben nach der Oberflächenmodifikation erhalten.

Poly-L-Ornithin und Poly-L-Lysin Lösungen wurden für 20min bei 37°C auf der Polymeroberfläche inkubiert; anschließend mit Phosphatpuffer (PBS) gespült. Fibronektin aus dem Rind wurde für 60 min bei 37°C inkubiert. Die Konzentration betrug 10 pg/ml PBS. Anschließend wurde mit PBS gewaschen und luftgetrocknet.

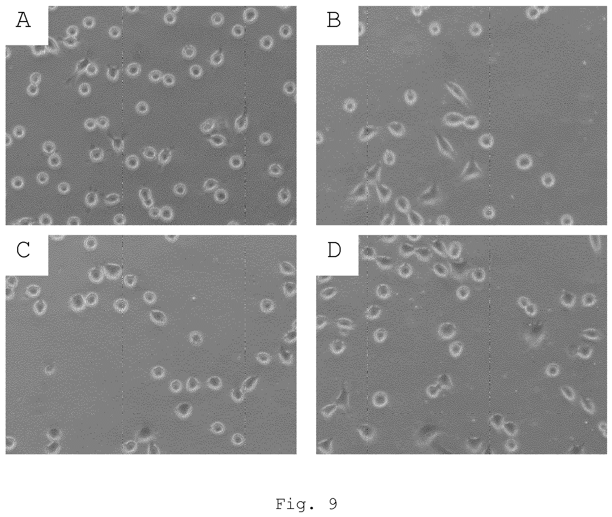

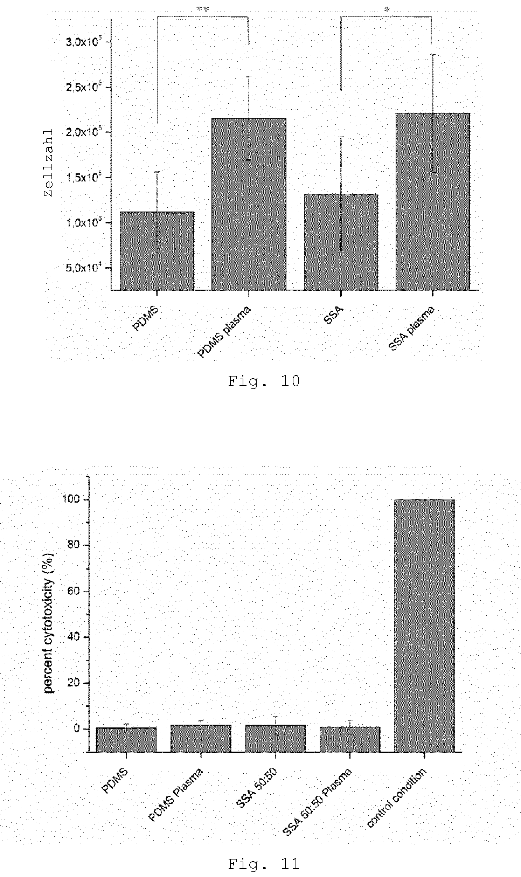

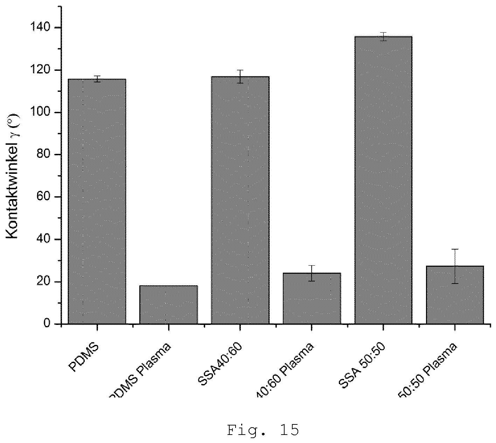

Außerdem wurde das Adhäsionsverhalten von L929 Zellen auf PDMS und SSA 50:50 nach 24h Kultivierungszeit untersucht. Dazu wurden 3x10⁵ vitale Zellen für 24h auf PDMS (A), SSA 50:50 (B) und plasmabehandeltem PDMS (C) und SSA 50:50 (D) kultiviert (Figur 9). Nach diesem Zeitraum wurden die Zellen enzymatisch von der Oberfläche entfernt und die Zellzahl bestimmt (Figur 10). Um einen zytotoxischen Effekt der Kultivierung auf PDMS oder SSA 50:50 zu untersuchen, wurde die Aktivität der Laktatdehydrogenase (LDH) nach 24h Kultivierung untersucht (Figur 11). Bei keiner Kondition konnte ein zytotoxischer Effekt beobachtet werden. Den Einfluss der Plasmabehandlung auf den Kontaktwinkel der Oberfläche zeigt Figur 15.

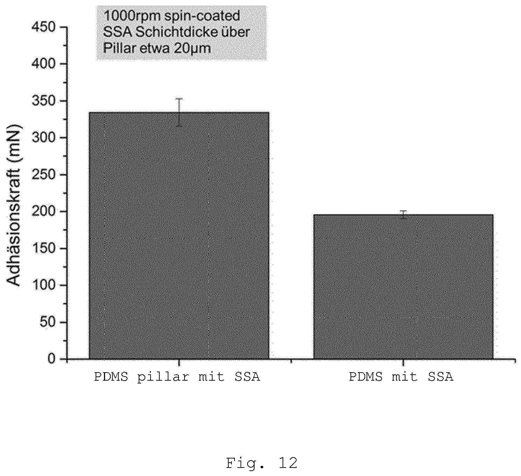

Den Einfluss der Strukturierung zeigt Figur 12. Mikrostrukturierte Oberflächen, die mit SSA 50:50 beschichtet wurden, beeinflussen die Adhäsionskraft. SSA 50:50 wurde mittels Spin-coating auf eine mikrostrukturierte PDMS Schicht mit Pillarhöhen von 20 µm aufgebracht. Die Adhäsionskraft bei den überschichteten Pillarstrukturen ist signifikant höher als in Bereichen, in denen keine Vorsprünge vorhanden sind. Die Messung wurde auf der gleichen Probe durchgeführt, welche strukturierte und unstrukturierte Bereiche aufwies.

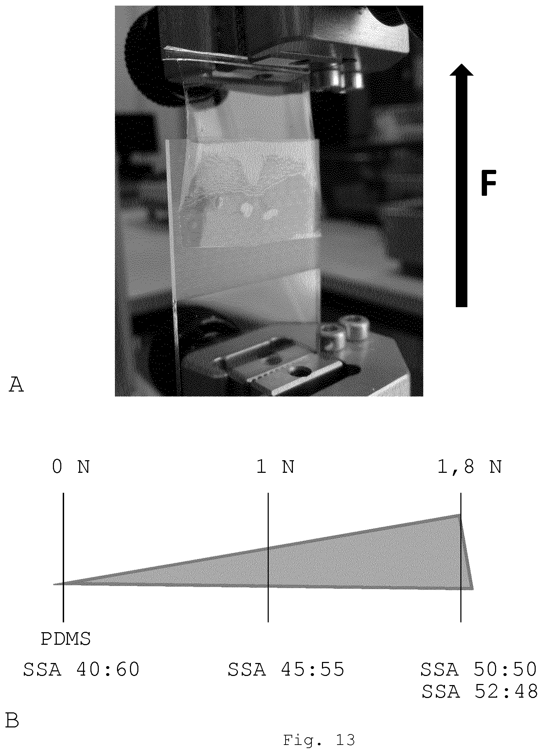

Figur 13 zeigt den Aufbau für Zugkraftversuche mit nichtstrukturierten Zweischichtkompositen gegen Vitro-Skin®. Dazu wurde ein Zweischichtkomposit bestehend aus einer PDMS Schicht, auf die SSA in den Mischungsverhältnissen 40:60 bis 52:48 aufgetragen wurde, hergestellt. Als Referenz diente ein Präparat aus PDMS. Figur 13 A zeigt exemplarisch den Aufbau des Versuches. Vitro-Skin® ist ein synthetisches Material, welches die Oberflächeneigenschaften menschlicher Haut simuliert (Rauigkeit Rₐ= 12 - 15 µm). PDMS und SSA im Mischungsverhältnis 40:60 zeigte keinerlei Haftung. Maximale Haftung konnte mit SSA in den Mischungsverhältnissen 50:50 und 52:48 erzielt werden (Figur 13 B).

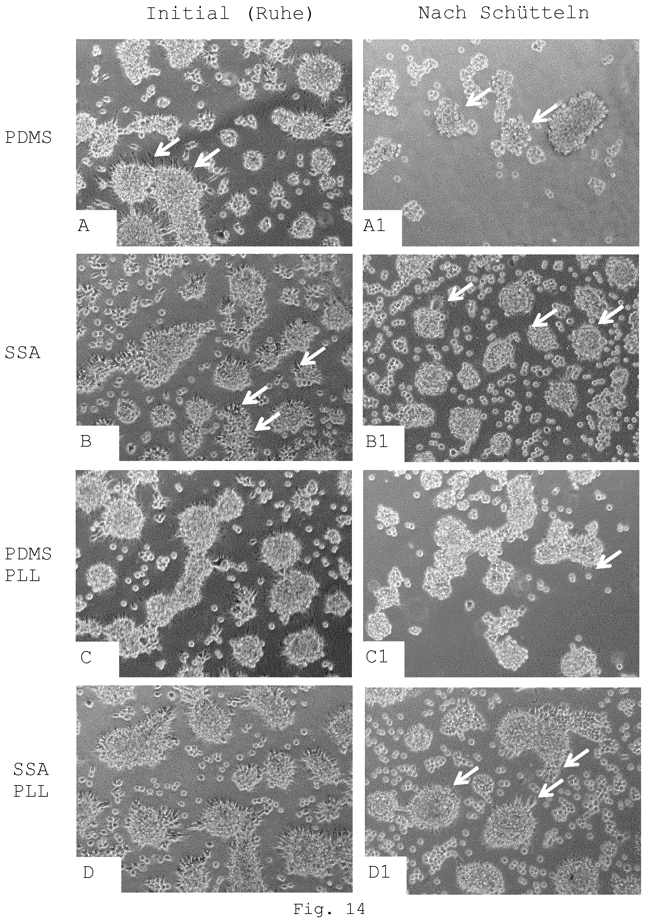

Die Adhäsionsstärke von L929-Zellen auf den Oberflächen wurde ebenfalls untersucht. Figur 14 zeigt entsprechende lichtmikroskopische Aufnahmen. L929-Zellen wurden für 48h auf PDMS (A) und SSA 50:50 (B) ausplattiert. Die mittlere Schichtdicke betrug zwischen 130 µm und 200 µm und wurde mit einem optischen System bestimmt.

Zusätzlich wurde die Oberfläche der Polymere durch das Aufbringen von 0,01% Poly-L-Lysin (PLL) funktionalisiert (PDMS (C) und SSA 50:50 (D)). Die Zellen wurden als Einzelzellen ausplattiert. Generell lässt sich mikroskopisch kein Unterschied in der quantitativen Adhäsion der Zellen zwischen PDMS und SSA beobachten, die Zellen bilden Fortsätze auf beiden Materialien aus (Pfeile in allen Abbildungen). Generell erscheinen die Zellen auf SSA abgeflachter und langgestreckter. Der gleiche Eindruck ist auf den PLL beschichteten Oberflächen zu erhalten (C,D). Um zu untersuchen, wie sich die Zellen nach einer mechanischen Belastung verhalten, wurden alle Proben für einen Zeitraum von etwa 60s mit gleicher Kraft geschüttelt. Dies führt zu einem signifikanten Ablösen der Zellen von der PDMS Oberfläche (A1). Die Aggregate in diesem Bild haben keinen Kontakt mehr zu der Polymeroberfläche (Pfeile in A1). Im Vergleich dazu findet sich auf der SSA Oberfläche eine deutliche Reduktion der zellulären Fortsätze im Vergleich mit A, allerdings findet kein Ablösen von der Oberfläche statt (B1). Die Funktionalisierung mittels PLL verhindert die Ablösung der Zellen auf der PDMS Oberfläche deutlich (C1) und verhindert die Reduktion der Zellfortsätze bei SSA (D1). Trotzdem erscheinen die Zellen "kugeliger" als in Abbildung D. Diese Morphologie erscheint typisch für Zellen mit geringen Adhäsionskontakten zur Oberfläche. Zusammenfassend lässt sich hier sagen, dass Zellen auf der SSA Oberfläche unempfindlicher gegen mechanische Belastung sind.

Die zelluläre Adhäsion lässt sich noch deutlich durch eine Oberflächenmodifikation verbessern, wie in D1 gezeigt ist.

SSA 50:50-PDMS Kompositstrukturen wurden hergestellt und auf das intakte Trommelfell einer toten Maus aufgebracht. Die Kompositstruktur wurde auf die benötigten Abmessungen zurechtgeschnitten und dann mit der klebenden Seite auf das intakte Trommelfell aufgebracht. Mehrmaliges Ablösen und repositionieren führte nicht zu einer Ruptur des Trommelfells. In einem weiteren Schritt wurde ein Teil des Trommelfells aufgeschnitten, um eine Ruptur zu simulieren. Die Kompositstruktur konnte an den Rändern der Wunde befestigt und ein lateraler Zug ausgeübt werden.

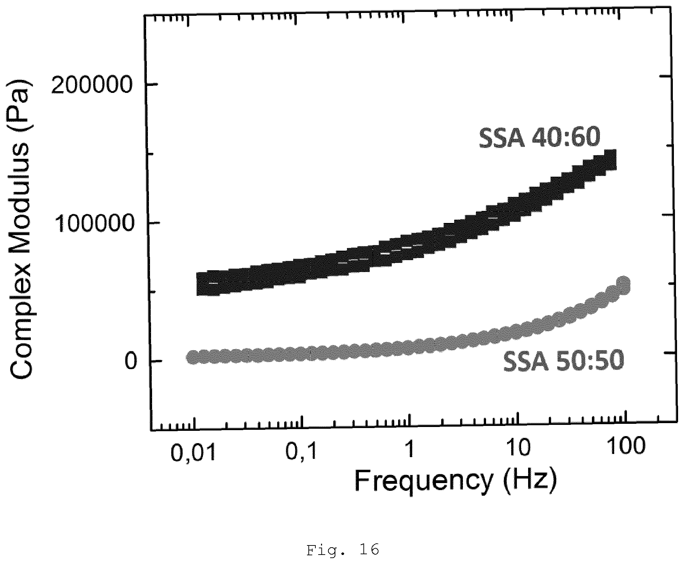

Das komplexe Modul von SSA 40:60 und SSA 50:50 wurde mittels Rheometrie mit Frequenzen zwischen 0,1 und 100 Hz ermittelt (Figur 16). Die Messamplitude betrug 0,1%. Die Ergebnisse zeigen, dass SSA 50:50 ein geringeres E-Modul als SSA 40:60 aufweist. Ungefähr ergibt sich für PDMS 10:1 und SSA 40:60 ein Verhältnis von etwa 6 und zwischen PDMS 10:1 und SSA 50:50 ein Verhältnis von etwa 65.

Im Vergleich beider Substrate zueinander zeigt sich, dass die Abzugsspannung von SSA bei Verwendung eines rauen Substrats (Glas Rₐ = 0,271 µm) höher ist, als bei PDMS (Figur 17). SSA 50:50 weist vergleichbare Abzugsspannungen auf, wenn ein raues oder glattes Substrat (Rₐ = 0,006 µm) verwendet wird (A, B). Die maximale Dehnung von SSA 50:50 ist wesentlich größer bei SSA 50:50, als bei PDMS (C). Die Adhäsionsenergie von PDMS ist wesentlich geringer bei Verwendung eines rauen Substrats, als die Adhäsionsenergie von SSA 40:60 und SSA 50:50 (D).

Dies zeigt, dass die erfindungsgemäßen strukturierten Beschichtungen, für raue Oberflächen besser geeignet sind, d.h. Oberflächen, welche eine Rauigkeit von über 0,2 µm aufweisen. Für ein Mäusetrommelfell wurde nach Aufdampfen eines dünnen Goldfilms eine Rauigkeit von ungefähr Rₐ = 1 µm gemessen.

Figur 18 zeigt in A eine schematische Darstellung der Messapparatur zur Ermittlung der Messwerte. Die strukturierte Beschichtung (polymer film) wird durch einen beweglichen Tisch (pivotable table) gegen ein Substrat (glass substrate) gedrückt. An dem Substrat kann über eine Kraftmessdose (load cell) die Andruckkraft und auch die Adhäsionskraft beim Wegbewegen der strukturierten Beschichtung gemessen werden. B zeigt die Messung der Rauigkeit der für die Messungen verwendeten Glasssubstrate (Rauigkeit immer gemessen mit Weißlichtinterferometrie).

Figur 19 zeigt den Herstellungsprozess. Dabei wird in (1) zunächst eine strukturierte Oberfläche hergestellt, welche dann in (2) zu einer strukturierten Beschichtung mit einer weiteren Schicht weiterverarbeitet wird. So zeigt (1) von links nach rechts das Aufbringen des ersten Polymers (prepolymer 1) auf entweder einen Glasobjektträger oder eine mikrostrukturierte MD40-Form (hexagonal angeordnete säulenförmige Vorsprünge mit 7 µm Durchmesser, 18 µm Höhe, Abstand Mittelpunkt zu Mittelpunkt 14 pm), die sich auf einem Glasobjektträger befindet. Neues PDMS wird auf die MD40 Form aufgebracht, im Vakuum wird Luft entzogen und ein plasmaaktivierter Glasobjektträger (plasma activated glass slide) auf die Oberfläche aufgebracht. Auf diesen werden Gewichte (apply weight) aufgebracht (z. B. 10 g/cm²), wodurch die Dicke der PDMS Schicht, welche die Trägerschicht für die Vorsprünge darstellt, beeinflussbar ist (z. B. 40 +/- 9 µm). Nach der Polymerisierung bei 95°C bis 100°C für eine Stunde kann die MD40 Form entfernt werden. (2) Auf die mikrostrukturierte PDMS Schicht, die noch mit Plasma behandelt wird, kann nun das zweite Polymer (prepolymer 2, z. B. SSA) mittels des spin-coating Verfahrens aufgebracht werden. Die nun hergestellte Kompositstruktur wird bei 95°C bis 100°C polymerisiert. Anschließend kann die Oberfläche noch für biologische Anwendungen funktionalisiert werden. Mit diesem Verfahren wurden sowohl strukturierte Beschichtungen als auch unstrukturierte Beschichtungen als Vergleichsproben hergestellt. Der Parameter t gibt die Dicke der weiteren Schicht oberhalb der Vorsprünge an, während b die Dicke der Trägerschicht angibt.

Figur 20 zeigt einen Querschnitt für weitere Ausführungsformen der Erfindung. So müssen die Vorsprünge im Längsschnitt nicht rechteckig geformt sein. Es ist auch möglich, dass die Stirnflächen der Vorsprünge gebogen sind, insbesondere konvex zur weiteren Schicht hin (Figur 20 oben).

Eine weitere Ausführungsform der Erfindung zeigt Figur 20 unten. Dort bildet die Grenzfläche zwischen Vorsprüngen und weitere Schicht eine Wellenlinie. Die Vorsprünge sind daher dreidimensional als Rotationsparaboloid geformt.

Wichtig für alle Varianten ist, dass es einen ausreichend großen Bereich der weiteren Schicht gibt, bei welchem die senkrechte Dicke der weiteren Schicht im erfindungsgemäßen Verhältnis in Bezug auf die Höhe der Vorsprünge in diesem Bereich vorliegt. In diesen Bereichen mit dünner weiteren Schicht kommt es zur Ausbildung von vorteilhaften Adhäsionseigenschaften. Durch die Vermeidung von Kanten bei der Form der Vorsprünge, insbesondere an den Stirnflächen, können Spannungsspitzen beim Ablösen der Vorrichtung von einer Oberfläche vermieden werden, was die Adhäsion verbessert.

### Bezugszeichen

- 100: Trägerschicht
- 110: Vorsprung
- 120: Weitere Schicht
- 130: Ausgefüllter Zwischenraum
- 140: Stirnfläche
- 150: Der Oberfläche des Substrats zugewandte Oberfläche

## Patentansprüche

1. Vorrichtung zur Verwendung bei der Behandlung von Trommelfellperforationen mit einer strukturierten Beschichtung, wobei die Vorrichtung eine Trägerschicht umfasst, wobei auf dieser Trägerschicht eine Vielzahl von Vorsprüngen angeordnet ist, die mindestens jeweils einen Stamm mit einer von der Oberfläche wegweisenden Stirnfläche umfassen, **dadurch gekennzeichnet, dass** mindestens auf der Stirnfläche eine weitere Schicht angeordnet ist, wobei diese Schicht einen anderen Elastizitätsmodul aufweist als der jeweilige Vorsprung und wobei die auf der Stirnfläche angeordnete weitere Schicht einen geringeren Elastizitätsmodul aufweist als der jeweilige Vorsprung.

2. Vorrichtung nach Anspruch 2, **dadurch gekennzeichnet, dass** die Vorsprünge ein Aspektverhältnis von größer 1 aufweisen.

3. Vorrichtung nach Anspruch 1 oder 2, **dadurch gekennzeichnet, dass** die Vorsprünge ein Aspektverhältnis von mindestens 3 aufweisen.

4. Vorrichtung nach einem der Ansprüche 1 bis 3, **dadurch gekennzeichnet, dass** die weitere Schicht zusätzlich die Zwischenräume zwischen den Vorsprüngen ausfüllt.

5. Vorrichtung nach einem der Ansprüche 1 bis 4, **dadurch gekennzeichnet, dass** die weitere Schicht Teil eines Films ist, welcher die Vorsprünge verbindet.

6. Vorrichtung nach einem der Ansprüche 1 bis 5, **dadurch gekennzeichnet, dass** die Vorrichtung zur Adhäsion auf weichen Substraten ausgebildet ist.

7. Vorrichtung nach einem der Ansprüche 1 bis 6, **dadurch gekennzeichnet, dass** die Vorrichtung zur Adhäsion auf biologischen Geweben ausgebildet ist.

8. Vorrichtung nach einem der Ansprüche 1 bis 7, **dadurch gekennzeichnet, dass** die kleinste Dicke der weiteren Schicht oberhalb eines Vorsprungs immer kleiner ist, als die maximale senkrechte Höhe des Vorsprungs.

9. Vorrichtung nach einem der Ansprüche 1 bis 8, **dadurch gekennzeichnet, dass** die senkrechte Höhe aller Vorsprünge in einem Bereich von 1 µm bis 10 mm liegt.

10. Vorrichtung nach einem der Ansprüche 1 bis 9, **dadurch gekennzeichnet, dass** die Elastizitätsmodule aller Bereiche des Vorsprungs und der weiteren Schicht bei 50 kPa bis 3 GPa liegen.

11. Vorrichtung nach einem der Ansprüche 1 bis 10, **dadurch gekennzeichnet, dass** die Vorsprünge aus Elastomeren bestehen.

12. Implantat umfassend eine Vorrichtung nach einem der Ansprüche 1 bis 11.

## Claims

1. A device for use in the treatment of eardrum perforations having a structured coating, wherein the device comprises a carrier layer, wherein a plurality of protrusions is arranged on this carrier layer, which protrusions each comprise at least a shaft having an end face pointing away from the surface, **characterized in that** a further layer is arranged at least on the end face, wherein this layer has a different elastic modulus than the protrusion in question and wherein the further layer arranged on the end face has a lower elastic modulus than the respective protrusion.

2. The device as claimed in claim 2, **characterized in that** the protrusions have an aspect ratio of greater than 1.

3. The device as claimed in claim 1 or 2, **characterized in that** the protrusions have an aspect ratio of at least 3.

4. The device as claimed in one of claims 1 through 3, **characterized in that** the further layer additionally fills the intermediate spaces between the protrusions.

5. The device as claimed in one of claims 1 through 4, **characterized in that** the further layer is part of a film that connects the protrusions.

6. The device as claimed in one of claims 1 through 5, **characterized in that** the device is configured for adhering to soft substrates.

7. The device as claimed in one of claims 1 through 6, **characterized in that** the device is configured for adhering to biological tissues.

8. The device as claimed in on of claims 1 through 7, **characterized in that** smallest thickness of the further layer above a protrusion is always less than the maximum perpendicular height of the protrusion.

9. The device as claimed in on of claims 1 through 8, **characterized in that** the perpendicular height of all of the protrusions is in a range of 1 µm to 10 mm.

10. The device as claimed in on of claims 1 through 9, **characterized in that** the elastic moduli of all areas of the protrusion and the further layer are 50 kPa to 3 GPa.

11. The device as claimed in on of claims 1 through 10, **characterized in that** are composed of elastomers.

12. An implant comprising a device as claimed in one of claims 1 through 11.

## Revendications

1. Dispositif destiné à être utilisé dans le traitement de perforations du tympan et pourvu d'un revêtement structuré, le dispositif comprenant une couche de support, une pluralité de saillies étant disposées sur cette couche de support, chacune d'elles comprenant au moins une tige pourvue d'une face frontale dirigée à l'opposé de la surface, **caractérisé en ce qu'**une autre couche est disposée au moins sur la face frontale, cette couche ayant un module d'élasticité différent de celui de la saillie respective et l'autre couche disposée sur la face frontale ayant un module d'élasticité inférieur à celui de la saillie respective.

2. Dispositif selon la revendication 2, **caractérisé en ce que** les saillies ont un rapport d'aspect supérieur à 1.

3. Dispositif selon la revendication 1 ou 2, **caractérisé en ce que** les saillies ont un rapport d'aspect d'au moins 3.

4. Dispositif selon l'une des revendications 1 à 3, **caractérisé en ce que** l'autre couche remplit en outre les espaces ménagés entre les saillies.

5. Dispositif selon l'une des revendications 1 à 4, **caractérisé en ce que** l'autre couche fait partie d'un film qui relie les saillies.

6. Dispositif selon l'une des revendications 1 à 5, **caractérisé en ce que** le dispositif est conçu pour adhérer à des substrats mous.

7. Dispositif selon l'une des revendications 1 à 6, **caractérisé en ce que** le dispositif est conçu pour adhérer à des tissus biologiques.

8. Dispositif selon l'une des revendications 1 à 7, **caractérisé en ce que** la plus petite épaisseur de l'autre couche au-dessus d'une saillie est toujours inférieure à la hauteur verticale maximale de la saillie.

9. Dispositif selon l'une des revendications 1 à 8, **caractérisé en ce que** la hauteur verticale de toutes les saillies est comprise entre 1 µm et 10 mm.

10. Dispositif selon l'une des revendications 1 à 9, **caractérisé en ce que** les modules d'élasticité de toutes les zones de la saillie et l'autre couche sont de 50 kPa à 3 GPa.

11. Dispositif selon l'une des revendications 1 à 10, **caractérisé en ce que** les saillies sont formées à partir d'élastomères.

12. Implant comprenant un dispositif selon l'une des revendications 1 à 11.
